# EUROPÄISCHE PATENTANMELDUNG

(11) **EP 1 531 152 A1**
(43) Veröffentlichungstag der Anmeldung: **18.05.2005**
(21) Anmeldenummer: 04105358.8
(22) Anmeldetag: 28.10.2004
(51) Int. Cl.: C07D 233/54, A61K 31/415, A61P 31/10

(54) **Verfahren zur Reinigung von Climbazol**

(30) Priorität: 17.11.2003 DE 10353673
(71) Anmelder: Symrise GmbH & Co. KG, 37603 Holzminden (DE)
(72) Erfinder: Körber, Alfred, 37603, Holzminden (DE); Michler, Michael, 37632, Eimen (DE); Rheinländer, Heinz-Dieter, 37639 Lütgenade (DE)
(74) Vertreter: Eisenführ, Speiser & Partner

(57) **Zusammenfassung**

Die Erfindung betrifft ein Verfahren zur Reinigung von Climbazol, sowie auf diese Weise hergestellte Climbazol-haltige Zusammensetzungen. Ausgehend von einem Gemisch enthaltend Climbazol und ein organisches Lösungsmittel wird Climbazol kristallisiert und das Lösungsmittel abgetrennt, um einen Kristallkuchen zu erhalten. Der Kristallkuchen wird wiederholt mit dem organischen Lösungsmittel gewaschen. Beim Kristallisieren und beim Waschen wird der Kristallkuchen nur so weit vom Lösungsmittel befreit wird, dass der Kristallkuchen noch vollständig mit dem Lösungsmittel benetzt ist.

## Beschreibung

Die vorliegende Erfindung betrifft ein Verfahren zur Reinigung von 1-(4-Chlorphenoxy)-1-(1H-imidazoyl)-3,3-dimethyl-2-butanon (Climbazol), sowie Zusammensetzungen enthaltend Climbazol: 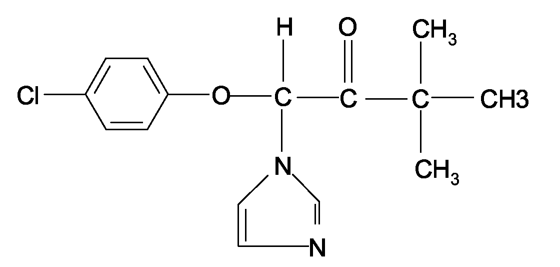 Climbazol wird wegen seiner antimykotischen Wirkung in kosmetischen Präparaten und insbesondere in Haarshampoos eingesetzt. Dementsprechend muss Climbazol in möglichst reiner Form vorliegen; insbesondere darf es nicht mit Begleitstoffen verunreinigt sein, die hautunverträglich sind, einen unangenehmen Geruch vermitteln oder eine unerwünschte Farbe verursachen.

In der DE 21 05 490 A werden drei Herstellungsverfahren für Imidazolderivate beschrieben, insbesondere auch für Climbazol. Zur Reinigung der hergestellten Imidazolderivate aus einer Lösung wird gemäß einer Verfahrensvariante a) gelehrt, das Lösungsmittel im Vakuum abzudampfen und den Rückstand mit einem polaren organischen Lösungsmittel aufzunehmen. Anschließend soll eine Wasserextraktion zum Entfernen des bei der Synthese entstehenden Nebenprodukts Imidazoylhydrochlorid durchgeführt werden, die Lösung soll danach bis zur Trocknung eingedampft werden. Aus dem Rückstand wird die Base durch Umkristallisieren, das Salz durch Behandeln mit der entsprechenden Säure nach üblichen Methoden gewonnen. Als Lösungsmittel bei der Herstellung der Imidazolderivate gemäß Verfahren a) können vorzugsweise polare organische Lösungsmittel verwendet werden, insbesondere Nitrile wie Azonitril, Sulfoxide wie Dimethylsulfoxid, Formamide wie Dimethylformamid, Ketone wie Aceton, Ether wie Diethylether und Tetrahydrofuran, Nitroalkane wie Nitromethan, und unsymmetrische Chlorkohlenwasserstoffe wie Methylenchlorid und Chloroform. In einem Beispiel zum Herstellen von Climbazol wird beschrieben, dass das Lösungsmittel einer Climbazol enthaltenden Lösung abdestilliert und der Rückstand drei Mal mit Wasser gewaschen wird. Anschließend wird der verbleibende Rückstand aus ungefähr 400 ml Ligroin umkristallisiert.

Zur Verfahrensvariante b) wird gelehrt, ein hochsiedendes organisches Lösungsmittel zu verwenden, insbesondere vorzugsweise aromatische Kohlenwasserstoffe wie z.B. Xylol, oder halogenierte aromatische Kohlenwasserstoffe wie z.B. Chlorbenzol. Mit Hilfe dieser Lösungsmittel kann entstehendes Reaktionswasser azeotrop abgetrennt werden. Die Verfahrensvariante b) kann auch ohne Lösungsmittel z.B. in einer Schmelze durchgeführt werden. Die Reinigung der gewünschten Imidazolderivate erfolgt nach üblichen Methoden.

In vergleichbarer Weise wird zur Verfahrensvariante c) gelehrt, beliebige inerte organische Lösungsmittel zu verwenden, vorzugsweise aromatische Kohlenwasserstoffe wie z.B. Benzol, Toluol, Ether wie z.B. Diethylether oder Tetrahydrofuran, chlorierte Kohlenwasserstoffe wie z.B. Methylenchlorid, Chloroform und Tetrachlorkohlenstoff, und niedere Alkylnitrile wie z.B. Acetonitril.

Wiederum erfolgt die Reinigung der gewünschten Imidazolderivate nach üblichen Methoden.

Aus EP 046 532 ist unter Beispiel a) ein Verfahren zur Herstellung des Climbazols aus 1-(4-Chlorphenoxy)-3,3-dimethyl-1-fluor-2-butanon mit Imidazol bekannt. Entsprechend den dort unter a) beschriebenen Verfahren werden beide Substanzen für 3 Stunden auf 130 °C erhitzt. Nach dem Erkalten wird der halbfeste Rückstand zwischen Methylenchlorid/Wasser verteilt, die organische Phase abgetrennt und eingeengt. Das zurückbleibende rohe Endprodukt wird über eine kleine Säule mit Siliciumdioxid chromatographiert.

In der DE 29 37 595 ist ebenfalls die Herstellung und Reinigung von Climbazol beschrieben: 4-Chlorphenol und Natriummethylat werden in Methanol mit 1-Brom-3,3-dimethyl-1-(imidazol-1-yl)-butan-2-on-hydrobromid umgesetzt. Der Rückstand wird zwischen Ether und Wasser verteilt, die organische Phase abgetrennt, mit Natronlauge, mit Wasser und mit Natriumchloridlösung gewaschen und über Natriumsulfat getrocknet. Nach Abdestillieren des Lösungsmittels wird der Rückstand aus Cyclohexan umkristallisiert. Die Ausbeute an Climbazol beträgt 70 % der Theorie; Schmelzpunkt 95 - 97° C.

Aufgrund der aus den Herstellungsprozessen resultierenden polaren und unpolaren Verunreinigungen wird Climbazol üblicherweise aus Isopropanol umkristallisiert. Dabei erhält man Climbazol in einer Ausbeute von ca. 80 % der Theorie in meist über 98 %iger Reinheit

Weniger polare Lösungsmittel wie z.B. Toluol liefern ca. 10 % bessere Ausbeute, jedoch ist das Endprodukt stets durch stark riechende Begleitstoffe, die u.a. aus Pivalinsäure bestehen, verunreinigt.

In der DE 197 03 651 wird die Schmelzkristallisation von Climbazol und weiterer Imidazole beschrieben. Die Kristallisation wird dabei an einer oder mehreren Kühlflächen durchgeführt, an denen Kristalle von Climbazol wachsen. Der Abkühlvorgang wird bei Erreichen einer gewünschten Kristallmasse beendet. Die nicht kristallisierte, an Climbazol ärmere Restflüssigkeit wird von den Kühlflächen bzw. den gebildeten Kristallen entfernt. Die an den Kühlflächen befindlichen Kristalle werden durch Temperaturanhebung an den Flächen abgeschmolzen. Die entstandene, an Climabazol angereicherte Flüssigkeit wird von den Flächen entfernt. Das Verfahren wird am Beispiel von 2-Methylimidazol explizit beschrieben. Ausgehend von einer 2-Methylimidazol-Qualität mit 99.1 Gew.-% Reinheit und 9000 ppm Verunreinigungen wird an einer Kühlfläche in einem Temperaturbereich zwischen 143 und 90 °C kristallisiert. Die gebildeten Kristalle werden bei einer Temperatur zwischen 143 und 160°C abgeschmolzen. Das abgeschmolzene Kristallisat weist einen Gehalt an 2-Methylimidazol von 99.9 Gew.-% und einen Gehalt an Verunreinigungen von 1000 ppm auf. Nachteilig an diesem Verfahren ist, dass die Verunreinigungen in der ersten Schmelzkristallisation nicht effizient genug abgetrennt werden können und mehrstufig gearbeitet werden muss.

In der DE 196 18 578 C1 wird ein Verfahren zur Reinigung von Climbazol beschrieben, bei dem auskristallisiertes Climbazol in Form eines Filterkuchens mit auf etwa 0 °C gekühltem Toluol gewaschen und das Toluol durch Beaufschlagen mit Stickstoff vom Feststoff abgedrückt wird. Anschließend wird eine weitere Wäsche mit ca. 5%iger Natriumhydrogencarbonat-Lösung durchgeführt. Schließlich wird der Filterkuchen in einem Trockner im Vakuum durch Mantelbeheizung getrocknet. Es wird eine Ausbeute von 90 % erreicht.

Nachteilig an diesem Verfahren ist, dass das kristalline Climbazol trotz Wäsche mit wässrigem Natriumhydrogencarbonat noch unerwünschte Begleitstoffe enthält, beispielsweise 400-700 ppm 4-Chlorphenol und einen starken synthesespezifischen Eigengeruch besitzt, der den Geruch kosmetischer Produkte stört. Die Begleitstoffe verursachen häufig insbesondere Hautreizungen oder sind unerwünscht allergen. Ferner zeigt großtechnisch hergestelltes Climbazol nach Lösung in organischen Lösungsmitteln wie Ethanol eine Trübung, die durch Restmengen von Natriumhydrogencarbonat verursacht wird, das nach Trocknen des feuchten Filterkuchens als Rückstand im Produkt verbleibt. Die Trübung stört bei der Verwendung in kosmetischen Produkten. Darüber hinaus besitzt das so hergestellte Climbazol häufig eine Hazen-Farbzahl von 400-500 (grau-weiße Farbe), was ebenfalls bei der Verwendung in kosmetischen Produkten störend auffällt.

Es war deshalb eine Aufgabe der vorliegenden Erfindung, ein Verfahren zum Aufarbeiten bzw. Reinigen von Climbazol anzugeben. Dabei sollten insbesondere häufig auftretende Begleitstoffe der Climbazol-Synthese so weit abgetrennt werden, dass sie bei der Verwendung des Climbazols in kosmetischen Produkten nicht mehr störend auffallen. Die betreffenden Begleitstoffe der Climbazol-Synthese sind: 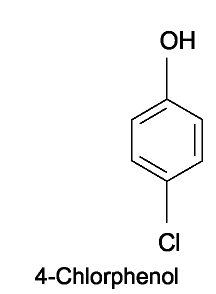 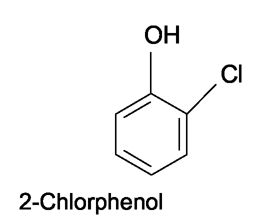 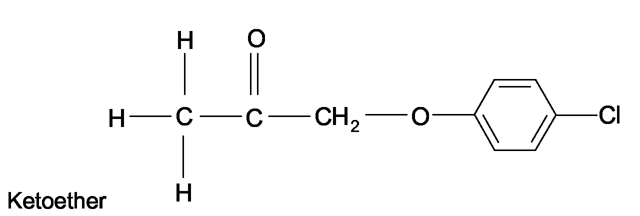 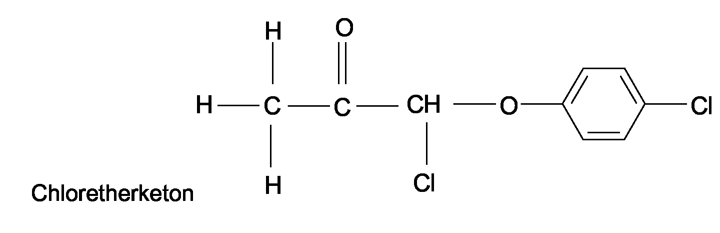 und

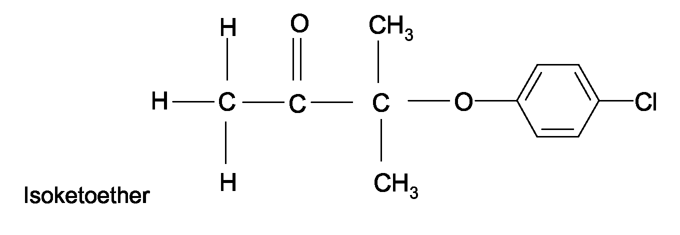

Insbesondere sollte der Gehalt an 4-Chlorphenol im gereinigten Climbazol auf ein für kosmetische Zwecke akzeptables Maß verringert werden. Ferner sollte das gereinigte Climbazol im wesentlichen geruchsneutral, weiß mit einer Hazen-Farbzahl < 300 (gemessen nach ISO 6271) und in Ethanol klar und ohne nennenswerte Trübung löslich sein.

Erfindungsgemäß wird deshalb ein Verfahren zum Reinigen von Climbazol angegeben, mit den Schritten:
a) ausgehend von einem Gemisch enthaltend Climbazol und ein organisches Lösungsmittel Kristallisieren von Climbazol und Abtrennen des Lösungsmittels, um einen Kristallkuchen zu erhalten, und
b) Waschen des so erhaltenen Kristallkuchens mit einem organischen Lösungsmittel, wobei der Kristallkuchen beim Kristallisieren und beim Waschen nur so weit vom Lösungsmittel befreit wird, dass der Kristallkuchen noch vollständig mit dem Lösungsmittel benetzt ist, und wobei Schritt b) wiederholt wird.

Wichtig für das verfahrensgemäße Reinigungsergebnis ist, dass der Kristallkuchen während des Waschens noch im wesentlichen vollständig mit Lösungsmittel benetzt und somit feucht bleibt. Der Kristallkuchen bleibt also sowohl beim Abtrennen vom Lösungsmittel in Schritt a) als auch beim Abtrennten des Lösungsmittels während des Waschens in Schritt b) mit dem Lösungsmittel noch im wesentlichen vollständig benetzt. Im Rahmen der vorliegenden Erfindung gilt der Kristallkuchen auch dann noch als vollständig mit einem Lösungsmittel benetzt, wenn ein Bereich weniger als 10 %, bevorzugt weniger als 5 % der von außen sichtbaren Oberfläche des Kristallkuchens nicht mit dem Lösungsmittel benetzt ist; besonders vorteilhaft ist die 100 %ige Benetzung des Kristallkuchens mit dem Lösungsmittel. Nach dem letzten Waschen am Ende des Verfahrens wird das Lösungsmittel vom Kristallkuchen so weit abgetrennt, dass dieser nicht mehr im wesentlichen vollständig mit dem Lösungsmittel benetzt ist. Zum Waschen in Schritt b) kann das gleiche organische Lösungsmittel wie zum Kristallisieren in Schritt a) verwendet werden. Ferner ist es bevorzugt, bei jedem Durchführen von Schritt b) das gleiche Lösungsmittel zu verwenden.

Es wurde gefunden, dass nach dem Auskristallisieren des Climbazols durch gezieltes Waschen mit dem Lösungsmittel in der soeben beschriebenen Weise eine Climbazol-enthaltende feste Zusammensetzung gewonnen werden kann, die folgende Eigenschaften besitzt:
- Gehalt an Climbazol: zumindest 98 Gew.-%, bezogen auf die Gesamtmenge an Feststoff,
- Gehalt an 4-Chlorphenol: nicht mehr als 400 ppm, vorzugsweise nicht mehr als 100 ppm, bezogen auf die Gesamtmenge an Feststoff, und
- Hazen-Farbzahl nicht mehr als 300, vorzugsweise nicht mehr als 200 (gemessen nach ISO 6271).

Die Zusammensetzung besteht daher im wesentlichen aus reinem Climbazol. Sie ist in Ethanol bis zur Sättigung klar löslich. Zudem besitzt sie in dem gegenüber herkömmlich erhältlichen Climbazol einen stark verringerten Eigengeruch, der ansonsten als unangenehm empfunden wird. Die erfindungsgemäß erhältliche Zusammensetzung ist deshalb vorteilhaft geeignet zur Verwendung in kosmetischen Präparaten, insbesondere Cremes und/oder Shampoos, wobei die Zusammensetzung geeignet ist zur Herstellung eines antimykotischen Arzneimittels und/oder antimykotischen kosmetischen Präparats, insbesondere einer Creme und/oder eines Shampoos.

Das erfindungsgemäße Verfahren ermöglicht es also erstmals, eine Zusammensetzung im wesentlichen reinen Climbazols herzustellen, die über die soeben beschriebenen vorteilhaften Eigenschaften verfügt. Zudem ist das erfindungsgemäße Verfahren leicht durchzuführen, es benötigt keine komplizierten Schmelzprozesse und liefert im wesentlichen reines Climbazol in hoher Ausbeute.

In Schritt a) des Verfahrens kann zweckmäßigerweise die Temperatur des auszukristallisierenden Gemischs auf -5 bis 10 °C, vorzugsweise auf 0-3 °C eingestellt werden. Bei diesen Temperaturen lässt sich Climbazol gut aus den bei der Synthese üblicherweise verwendeten organischen Lösungsmitteln auskristallisieren. Alternativ oder zusätzlich dazu können Climbazol-Kristalle zum Animpfen des Kristallisationsvorgangs hinzugefügt werden.

Der Schritt b) des erfindungsgemäßen Verfahrens wird zumindest einmal wiederholt. So lässt sich besonders einfach und sicher die Zusammensetzung im wesentlichen reinen Climbazols mit den zuvor beschriebenen vorteilhaften Eigenschaften herstellen. Im Allgemeinen genügt es, wenn Schritt b) zwei Mal durchgeführt wird, wobei eine besonders gute Reinheit der Zusammensetzung erreicht wird, wenn Schritt b) drei Mal durchgeführt wird. In manchen Fällen mag eine bis zu viermalige Durchführung des Schrittes b) von Vorteil sein.

Zum Waschen wird zweckmäßigerweise ein solches Lösungsmittel verwendet, das im wesentlichen frei ist an Begleitstoffen, die im gereinigten Climbazol unerwünschte Begleitstoffe wären und insbesondere dessen Verwendung in Kosmetika durch Vermitteln eines unangenehmen Geruchs, einer unerwünschten Farbe oder durch Verursachen von Hautreizungen oder Auslösen von Allergien beeinträchtigen können. Das Lösungsmittel ist deshalb vorzugsweise frei von den oben aufgezählten Begleitstoffen der Climbazol-Synthese.

Ein zum Waschen besonders geeignetes organisches Lösungsmittel ist ausgewählt aus der Gruppe bestehend aus aromatischen Kohlenwasserstoffen, gesättigten Kohlenwasserstoffen und polaren organischen Lösungsmitteln, oder ist eine Mischung von zwei oder mehr Mitgliedern dieser Gruppe. In besonders bevorzugten Ausführungsformen ist das organische Lösungsmittel ausgewählt aus der Gruppe bestehend aus Benzol, Toluol, Xylol, Ethylbenzol, ein Alkan mit bis zu 12 Kohlenstoffatomen, Methanol, Ethanol, Isopropanol, Aceton, Cyclohexanon, Pentan, Hexan, Heptan, Octan, Isooctan, Cyclohexan, Methylcyclohexan und Decalin oder ist eine Mischung aus zwei oder mehr Mitgliedern dieser Gruppe. Vorteilhafterweise beträgt der Wassergehalt im Lösungsmittel kleiner oder gleich 5 Gew.-%, bevorzugt kleiner oder gleich 2 Gew.-%. Das bevorzugte Lösungsmittel ist Toluol mit zumindest technischer Reinheit; dieses Lösungsmittel ermöglicht eine hohe Climbazol-Ausbeute bei hoher Reinheit des erhaltenen Climbazols.

Zur Durchführung des erfindungsgemäßen Verfahrens ist es bevorzugt, wenn in Schritt b) der Kristallkuchen zum Waschen mit dem Lösungsmittel in einem Volumenverhältnis von Lösungsmittel zu Kristallkuchen von 0,5:1 bis 5:1, bevorzugt 0,8:1 bis 1,5:1 beaufschlagt wird. Das Beaufschlagen erfolgt bevorzugt durch Überschichten des Kristallkuchens mit dem Lösungsmittel. Der Kristallkuchen wird vorzugsweise mit einer Lösungsmittelmenge von einfacher bis doppelter Schichtdicke des Kristallkuchens überschichtet. Bei Einhalten der beschriebenen Lösungsmittelmengen zum Waschen des Kristallkuchens werden besonders reine Climbazol-enthaltende Zusammensetzungen in hoher Ausbeute gewonnen.

Ferner ist es bevorzugt, den Kristall kuchen nach dem Abtrennen des Lösungsmittels in Schritt a) und/oder b) glattzustreichen. Dies kann zweckmäßigerweise mit den Blättern eines Rührers geschehen. Durch das Glattstreichen wird ein Kristallkuchen von gleichmäßiger Stärke erzeugt. Der so behandelte Kristallkuchen erleichtert es, das Lösungsmittel in einem nachfolgenden Waschschritt so zu entfernen, dass der Kristallkuchen insgesamt gleichmäßig mit dem Lösungsmittel benetzt ist. Zudem können durch das Glattstreichen ein Bereich des Kristallkuchens von nicht mehr als 10 % der von außen sichtbaren Kristallkuchen-Oberfläche, der nach dem Abtrennen des Lösungsmittels nicht mehr mit dem Lösungsmittel benetzt sind, mit benetzten Bereichen des Kristallkuchens in Kontakt gebracht und auf diese Weise wieder mit Lösungsmittel benetzt werden, so dass der Kristallkuchen im Ergebnis im wesentlichen gleichmäßig mit Lösungsmittel benetzt ist.

Dementsprechend ist es auch bevorzugt, das Climbazol-enthaltende Gemisch während des Abtrennens des Lösungsmittels in Schritt a) zu rühren. Auf diese Weise wird die Bildung eines gleichmäßig starken Kristallkuchens vorteilhaft unterstützt.

Besonders bevorzugt sind solche Ausführungsformen des erfindungsgemäßen Verfahrens, bei denen der Kristallkuchen zum Durchführen von Schritt b) auf einem Filter verteilt und das Lösungsmittel in Schritt a) und/oder in Schritt b) durch Gas-Druckbeaufschlagung ohne Gasdurchbruch abgedrückt wird. Der Kristallkuchen bildet bei dieser Ausführungsform einen Filterkuchen auf dem Filter. Auf diese Weise kann das Lösungsmittel besonders leicht entfernt werden. Zudem kann durch Einstellen des Gasdrucks und der Dauer der Gasbeaufschlagung besonders einfach sichergestellt werden, dass der Kristallkuchen nach Abtrennen des Lösungsmittels noch vollständig mit dem Lösungsmittel benetzt ist. Als Gase zur Gas-Druckbeaufschlagung werden vorteilhafterweise inerte Gase wie beispielsweise Druckluft, Argon, Stickstoff eingesetzt, besonders bevorzugt ist Stickstoff. Vorteilhafterweise beträgt der Überdruck des Gases während der Gas-Druckbeaufschlagung 0,5 bis 2 bar, vorzugsweise 0,8 bis 1,2 bar, besonders bevorzugt 1 bar.

Die Temperatur des organischen Lösungsmittels wird in bevorzugten Ausführungsformen des erfindungsgemäßen Verfahrens zu Beginn von Schritt b) eingestellt auf -5 bis 5°C, vorzugsweise 0-3°C. Mit Lösungsmitteln bei diesen Temperaturen lassen sich hohe Ausbeuten und eine gute Reinheit der Climbazolenthaltenden Zusammensetzung erreichen.

Soweit zuvor nicht ausdrücklich etwas Gegenteiliges gesagt wurde, können die beschriebenen Ausführungsformen des erfindungsgemäßen Verfahrens beliebig miteinander kombiniert werden. Insbesondere ist zum Reinigen von Climbazol ein Verfahren mit den folgenden Schritten bevorzugt:
- ausgehend von einem Gemisch enthaltend Climbazol und ein organisches Lösungsmittel, vorzugsweise Toluol und/oder n-Heptan, Kristallisieren von Climbazol,
- Verteilen des Gemischs auf einem Filter und Abtrennen des Lösungsmittels durch Gas-Druckbeaufschlagung mit 0,8 bis 1,2 bar Stickstoff ohne Gasdurchbruch, um einen Kristallkuchen zu erhalten, und
- zweimaliges Waschen des so erhaltenen Kristallkuchens mit einem organischen Lösungsmittel, vorzugsweise Toluol und/oder n-Heptan, wobei der Kristallkuchen mit einer Lösungsmittelmenge von einfacher bis doppelter Schichtdicke des Kristallkuchens überschichtet wird, und das Lösungsmittel abgetrennt wird durch Gas-Druckbeaufschlagung mit 0,8 bis 1,2 bar Stickstoff ohne Gasdurchbruch.

Die Erfindung wird nachfolgend anhand der Beispiele näher beschrieben:

### Beispiel 1: Kristallisation und Reinigung von Climbazol in Toluol

In einem 5 m³ - Lösebehälter werden 2.500,-kg Toluol vorgelegt und über eine Feststoffbefüllung 3.000,- kg 92%iges Climbazol roh zugegeben. Die Suspension wird auf 80 - 85°C aufgeheizt, wobei sich das rohe Climbazol löst. Die Lösung wird über einen Filter umgepumpt, um Spuren von nicht löslichen Feststoffen zu entfernen. Das klare Filtrat wird in einen 5 m³ - Kristallisierbehälter gepumpt.

Im Kristallisierbehälter wird durch Animpfen mit Climbazol-Kristallen und langsames Abkühlen die Lösung auf 0 - 3°C eingestellt. Die Kristallsuspension wird unter Rühren in einen auf 0°C gekühlten Filtertrockner gepumpt. Der Rührer wird auf eine so niedrige Rührgeschwindigkeit gestellt, dass sich ein gleichmäßig starker Filterkuchen ausbilden kann.

Die Flüssigphase über dem Filterkuchen wird mit 1 bar Stickstoffüberdruck nur soweit abgedrückt, dass kein Gasdurchbruch erfolgt und der verbleibenden Kristallkuchen mit Lösungsmittel benetzt bleibt. Danach wird der Überdruck entspannt. Der Filterkuchen wird mit den Rührerblättern glattgestrichen, um eventuell entstandene Risse im Filterkuchen zu schließen. Der Rührer wird anschließend abgestellt.

Der Kristallkuchen wird mit 1.000 Liter 0 - 3°C kaltem Toluol überschichtet. Die Toluolschicht wird erneut mit 1 bar Stickstoffüberdruck abgedrückt, ohne dass ein Gasdurchbruch erfolgt.

Der letzte Vorgang wird noch 2 Mal mit 600 Litern Toluol wiederholt, wobei beim letzten Spülen des Filterkuchens bis zum Gasdurchbruch und 30 Minuten darüber hinaus abgedrückt wird.

Danach wird unter Rühren und Anlegen von Vakuum und Heizen der Filterkuchen im gleichen Apparat getrocknet.
Ausbeute: 2.580,-kg = 93,5% der Theorie
Reinheit des Climbazol: 99,1%

Man erhält ein weißes Kristallisat, frei von Chlorphenolen und geruchlich neutral. Das Kristallisat löst sich klar und rückstandsfrei in Ethanol.

### Beispiel 2: Kristallisation und Reinigung von Climbazol in n-Heptan

In einem 5 m³ - Lösebehälter werden 3.000,- kg n-Heptan vorgelegt und über eine Feststoffbefüllung 1.500,- kg 92%iges Climbazol roh zugegeben. Die Suspension wird auf 80 - 85 °C aufgeheizt, wobei sich das rohe Climbazol löst. Die Lösung wird über einen Filter umgepumpt, um Spuren von nicht löslichen Feststoffen zu entfernen. Das klare Filtrat wird in einen 5 m³ - Kristallisierbehälter gepumpt.

Im Kristallisierbehälter wird durch Animpfen mit Climbazol-Kristallen und langsames Abkühlen die Lösung auf 0 - 3°C eingestellt. Die Kristallsuspension wird wie im Beispiel 1 mit 700 Litern und 2 Mal mit 400 Litern n-Heptan durch Verdrängungswäsche gewaschen.
Ausbeute: 1.290,-kg = 92,4% der Theorie
Reinheit des Climbazol: 99,0%

Man erhält ein weißes Kristallisat, frei von Chlorphenolen und geruchlich neutral. Das Kristallisat löst sich klar und rückstandsfrei in Ethanol.

### Beispiel 3: Kristallisation und Reinigung von Climbazol in Isopropanol

In einen 500 ml Glaskolben werden 200 g Isopropanol und 300 g 92%iges Climbazol roh zugegeben. Der Kolben wird zum Rückflusssieden erhitzt. Die Lösung wird in einem Eisbad langsam auf 0 - 3 °C gekühlt. Die ausgefallenen Kristalle werden über einer Nutsche vom Lösungsmittel getrennt.
Der Filterkuchen wird noch 2 Mal mit 0°C kaltem Isopropanol in der in Beispiel 1 und 2 angegebenen Weise durchspült, die erste Verdrängungswäsche wurde mit 100 ml Isopropanol, die zweite mit 60 ml Isopropanol durchgeführt.
Ausbeute: 240g = 87,3% der Theorie
Reinheit des Climbazol: 99,3%

Man erhält ein weißes Kristallisat, frei von Chlorphenolen und geruchlich neutral. Das Kristallisat löst sich klar und rückstandsfrei in Ethanol.

### Beispiel 4: Übersicht verschiedener erfindungsgemäß hergestellter Climbazol-Zusammensetzunqen

| Probe | Gehalt Climbazol [%] | Gehalt 2-Chlorphenol [%] | Gehalt 4-Chlorphenol [%] | Gehalt 2,4-Dichlorphenol [%] | Gehalt 3,4-Dichlorphenol [%] | Hazen-Farbzahl | Geruch |
|---|---|---|---|---|---|---|---|
| 1 | **99,5** | 0 | 0,003 | 0 | 0 | **158** | A-B |
| 2 | **98,7** | 0 | 0,002 | 0 | 0 | **193** | A-B |
| 3 | **99** | 0 | 0,002 | 0 | 0 | **198** | A-B |
| 4 | **98,1** | 0 | 0,004 | 0 | 0 | **202** | A-B |
| 5 | **98,1** | 0 | < 0,001 | 0 | 0 | **178** | A-B |

Geruchsbewertung: A: Geruchlich gut; B: Geruch akzeptabel; C: Geruch inakzeptabel

### Vergleichsbeispiel: Übersicht herkömmlicher Climbazol-Zusammensetzungen

| Probe | Gehalt Climbazol [%] | Gehalt 2-Chlorphenol [%] | Gehalt 4-Chlorphenol [%] | Gehalt 2,4-Dichlorphenol [%] | Gehalt 3,4-Dichlorphenol [%] | Hazen-Farbzahl | Geruch |
|---|---|---|---|---|---|---|---|
| 1 | **99,4** | 0 | 0,045 | 0 | 0 | **504** | C |
| 2 | **99,1** | 0 | 0,063 | 0 | 0 | **442** | C |

## Patentansprüche

1. Verfahren zum Reinigen von Climbazol, umfassend die Schritte:
a) ausgehend von einem Gemisch enthaltend Climbazol und ein organisches Lösungsmittel Kristallisieren von Climbazol und Abtrennen des Lösungsmittels, um einen Kristallkuchen zu erhalten, und
b) Waschen des so erhaltenen Kristallkuchens mit einem organischen Lösungsmittel,
**dadurch gekennzeichnet, dass**
- der Kristallkuchen beim Kristallisieren und beim Waschen nur so weit vom Lösungsmittel befreit wird, dass der Kristallkuchen noch vollständig mit dem Lösungsmittel benetzt ist, und dass
- Schritt b) wiederholt wird.

2. Verfahren nach Anspruch 1, **dadurch gekennzeichnet, dass** das zum Waschen verwendete organische Lösungsmittel ausgewählt ist aus der Gruppe bestehend aus aromatischen Kohlenwasserstoffen, gesättigten Kohlenwasserstoffen und polaren organischen Lösungsmitteln, oder eine Mischung von zwei oder mehr Mitgliedern dieser Gruppe ist.

3. Verfahren nach Anspruch 2, **dadurch gekennzeichnet, dass** das zum Waschen verwendete organische Lösungsmittel ausgewählt ist aus der Gruppe bestehend aus Benzol, Toluol, Xylol, Ethylbenzol, ein Alkan mit bis zu 12 Kohlenstoffatomen, Methanol, Ethanol, Isopropanol, Aceton, Cyclohexanon, Pentan, Hexan, Heptan, Octan, Isooctan, Cyclohexan, Methylcyclohexan und Decalin, oder eine Mischung von zwei oder mehr Mitgliedern dieser Gruppe ist.

4. Verfahren nach einem der vorigen Ansprüche, **dadurch gekennzeichnet, dass** zum Waschen in Schritt b) der Kristallkuchen mit dem Lösungsmittel in einem Volumenverhältnis von Lösungsmittel zu Kristallkuchen von 0,5:1 bis 5:1, bevorzugt 0,8:1 bis 1,5:1 beaufschlagt wird.

5. Verfahren nach einem der vorigen Ansprüche, **dadurch gekennzeichnet, dass** der Kristallkuchen nach dem Abtrennen des Lösungsmittels glattgestrichen wird.

6. Verfahren nach einem der vorigen Ansprüche, **gekennzeichnet durch** Rühren während des Abtrennens des Lösungsmittels in Schritt a).

7. Verfahren nach einem der vorigen Ansprüche, **dadurch gekennzeichnet, dass** der Kristallkuchen zum Durchführen von Schritt b) auf einem Filter verteilt und das Lösungsmittel in Schritt a) und/oder in Schritt b) durch Gas-Druckbeaufschlagung ohne Gasdurchbruch abgedrückt wird.

8. Verfahren nach einem der vorigen Ansprüche, **dadurch gekennzeichnet, dass** die Temperatur des organischen Lösungsmittels zu Beginn von Schritt b) eingestellt wird auf -5 bis 5°C, vorzugsweise 0-3°C.

9. Climbazolenthaltende Zusammensetzung, herstellbar durch ein Verfahren nach einem der vorherigen Ansprüche.

10. Climbazolenthaltende Zusammensetzung, mit den Eigenschaften:
- Gehalt an Climbazol: zumindest 98 Gew.-%, bezogen auf die Gesamtmenge an Feststoff,
- Gehalt an 4-Chlorphenol: nicht mehr als 400 ppm, bezogen auf die Gesamtmenge an Feststoff, und
- Hazen-Farbzahl nicht mehr als 300.

11. Verwendung einer Climbazol enthaltende Zusammensetzung nach einem der Ansprüche 9 oder 10 zum Herstellen eines Arzneimittels und/oder kosmetischen Präparats.
